Europäisches Patentamt

European Patent Office

Office européen des brevets

Veröffentlichungsnummer: **0 283 932**

A2

# EUROPÄISCHE PATENTANMELDUNG

Anmeldenummer: 88104248.5

Anmeldetag: **17.03.88**

Int. Cl.⁴: **C12N 15/00** , C12P 21/02 , A61K 37/64 , C07K 7/10 , C07K 7/14 , C07K 13/00 , C12N 1/20 , C12N 5/00 , C12Q 1/68

Patentansprüche für folgende Vertragsstaaten:
ES + GR.

Priorität: **20.03.87 DE 3709255**

Veröffentlichungstag der Anmeldung:
**28.09.88 Patentblatt 88/39**

Benannte Vertragsstaaten:
**AT BE CH DE ES FR GB GR IT LI LU NL SE**

Anmelder: **HOECHST AKTIENGESELLSCHAFT**
**Postfach 80 03 20**
**D-6230 Frankfurt am Main 80(DE)**

Erfinder: **Ragg, Hermann, Dr.**
**Am Kirchplatz 14**
**D-6233 Keikheim (Taunus)(DE)**
Erfinder: **Preibisch, Gerald, Dr.**
**Johann-Strauss-Strasse 18**
**D-6233 Keikheim (Taunus)(DE)**
Erfinder: **Hein, Friedrich, Dr.**
**Hufnagelstrasse 13**
**D-8000 München 21(DE)**
Erfinder: **Uhlmann, Eugen, Dr.**
**Zum Talblick 31**
**D-6246 Glashütten Taunus(DE)**
Erfinder: **Lindenmaier, Werner, Dr.**
**Treppenkamp 10**
**D-3320 Salzgitter-Ringelheim(DE)**

Hybridserpine und dafür kodierende DNA.

Das humane Leuserpin-2 (hLS2) weist die gleiche Genstruktur wie α-Antitrypsin oder Angiotensinogen auf. Durch Kombination von Exons aus Serpingenen, die diese Genstruktur aufweisen, lassen sich rekombinante Gene herstellen, die für Hybridserpine kodieren. Durch Expression der rekombinanten Gene in eukaryotischen Zellen lassen sich die entsprechenden Hybridserpine gewinnen.

EP 0 283 932 A2

## Hybridserpine und dafür kodierende DNA

Aus der europäischen Patentanmeldung mit der Veröffentlichungs-Nr. (EP-A) 0 190 652 ist ein humanes Serpin bekannt, das inzwischen die Bezeichnung "Leuserpin-2" (hLS2) erhalten hat. In dieser EP-A ist weiterhin die cDNA für hLS2 wiedergegeben und auf die Möglichkeit verwiesen, modifizierte Gene oder Teilgene herzustellen, die zu entsprechend modifizierten Proteinen führen, in denen einzelne Aminosäuren ausgetauscht, weggelassen oder eingefügt werden. Es wird beispielsweise ein "Baukasten-Prinzip" erwähnt, bei dem ein Teilgen I, welches für die Aminosäurefolge vor dem aktiven Zentrum kodiert mit einem Teilgen II, welches für die Aminosäurefolge nach dem aktiven Zentrum kodiert, unter Zwischenschaltung eines Genfragmentes, welches für ein aktives Zentrum kodiert, kombiniert wird.

Es wurde nun der genomische Klon gefunden, der für hLS2 kodiert, und dessen Genstruktur ermittelt. Dieser Genaufbau wird erfindungsgemäß zur Herstellung neuer Hybridserpine genutzt.

Die Serpine sind eine Gruppe von Proteinen, die Funktionen als Proteinase-Inhibitoren bei der Blutgerinnung, der Komplementaktivierung und bei verschieden Aspekten der Entzündungsreaktionen ausüben. Die Serpine gehören einer Proteinfamilie an, deren Mitglieder eine Aminosäurehomologie von etwa 20 bis 35 % zueinander haben (R.F. Doolittle, Science 222 (1983) 417-419; H. Ragg, Nucl. Acids. Res. 14 (1986) 1073-1088). Die Spezifität dieser Proteinase-Inhibitoren wird einerseits durch eine Aminosäure in der P1-Position des reaktiven Zentrums bestimmt (M. Laskowski und I. Kato, Annu. Rev. Biochem. 49 (1980) 593-629), andererseits aber auch durch weitere Aminosäuresequenzen und Strukturelemente, die offenbar auch einen Einfluß auf die Aktivität der Serpine ausüben. Überdies spielt bei einigen Serpinen, wie z.B. bei Angiotensinogen, der N-terminale Bereich eine eigenständige funktionelle und strukturelle Rolle (S. Synder und R. Innis, Annu. Rev. Biochem. 48 (1979) 755-782).

Überraschenderweise liegt der untereinander ähnlichen Primär-und Tertiär-Struktur der Serpine (H. Loebermann et al., J. Mol. Biol. 177 (1984) 531-556; S.C. Bock et al. Biochemistry 25 (1986) 4292-4301) keine einheitliche Genstruktur zugrunde, wie das bei vielen anderen Proteinfamilien, beispielsweise bei den Globinen, der Fall ist. Von den bisher beschriebenen Serpingenen weisen nur diejenigen für humanes $\alpha^1$-Antitrypsin und für Ratten-Angiotensinogen äquivalente Exon-Intron-Strukturen auf (T. Tanaka et al., J. Biol. Chem. 259 (1984) 8063-8065): Jeweils 5 Exons werden an korrespondierenden Positionen von vier Introns unterbrochen. Die Struktur der übrigen bisher bekannten Serpingene unterscheidet sich jedoch wesentlich von diesem Muster. Das humane Antithrombin-III-Gen enthält 6 Exons (E.V. Prochownik et al., J. Biol. Chem. 260 (1985) 9608-9612), jedoch läßt sich nur eines der 5 Introns mit einer der Intronpositionen von $\alpha^1$-Antitrypsin bzw. Angiotensinogen homologisieren. Das Gen für den humanen C1-Inhibitor weist mindestens 7 Introns auf, deren Lokalisation jedoch noch nicht bekannt ist (S.C. Bock et al., a.a.O.).

Es wurde nun gefunden, daß die Gen-Struktur von hLS2 derjenigen des $\alpha^1$-Antitrypsins und des (Ratten-) Angiotensinogens hinsichtlich der Anzahl und Lokalisation der Introns entspricht. Diese Analogie wird erfindungsgemäß zur Herstellung der Hybridserpine genutzt.

Die Erfindung ist in ihren verschiedenen Aspekten in den Patentansprüchen definiert. Ausgestaltungen der Erfindung und bevorzugte Ausführungsformen werden im folgenden erläutert.

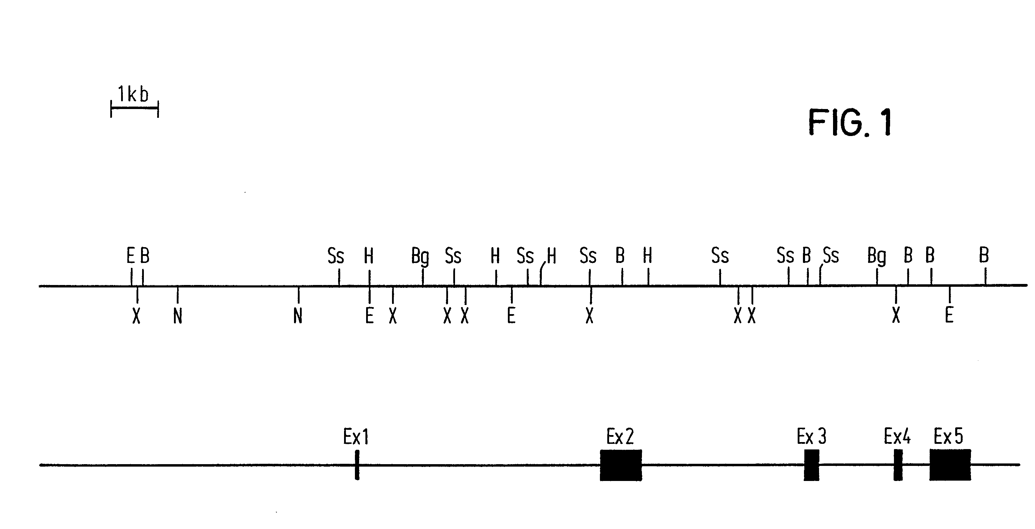

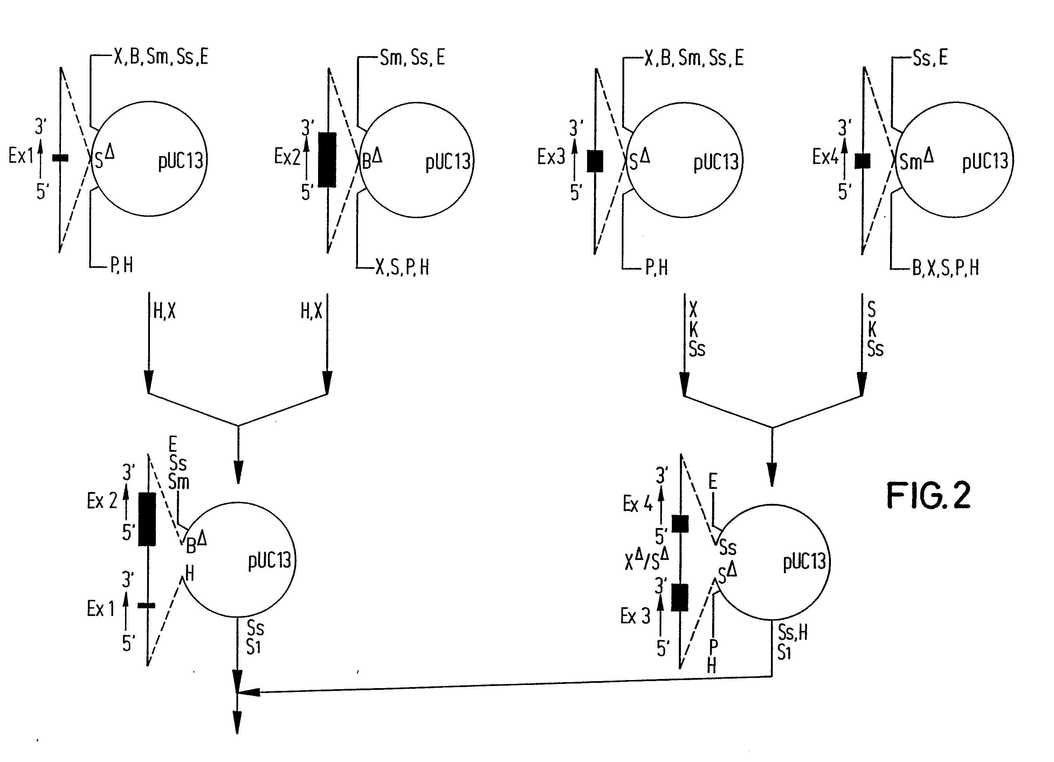

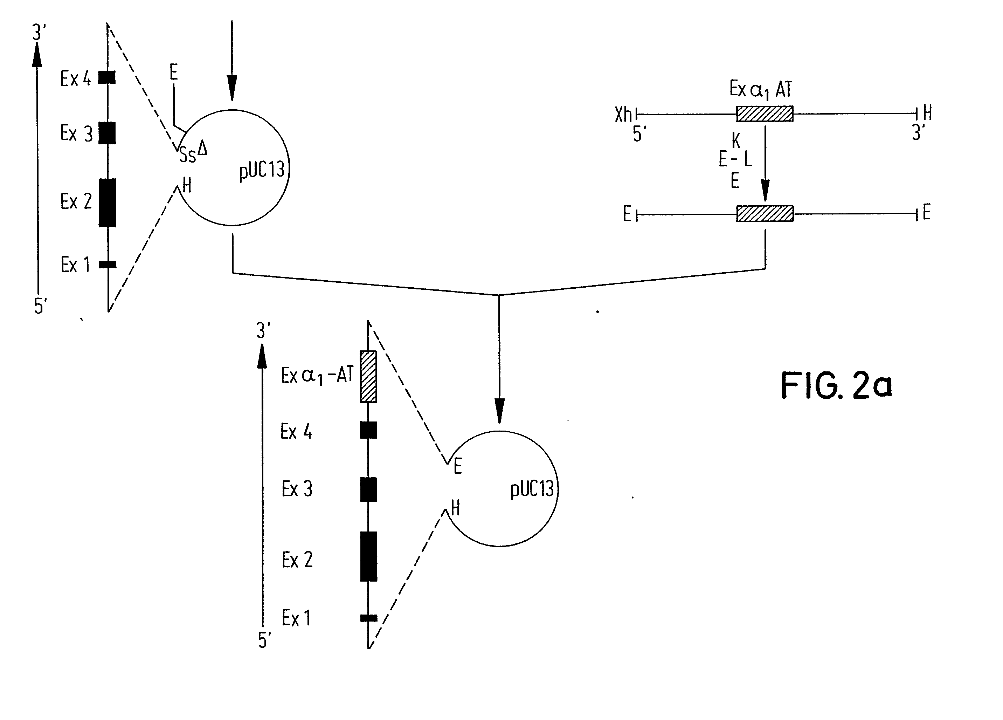

Die Erfindung ist weiterhin in den Figuren 1 und 2 sowie in der Tabelle 1 (Anhang) dargestellt (wobei hier und im folgenden unter "Figur 2" auch deren Fortsetzung in Figur 2a verstanden wird):

Die Figur 1 zeigt die Struktur des hLS2-Gens schematisch. "Ex 1" bis "Ex 5" stellen die Exons dar. Die Restriktionsschnittstellen sind wie folgt abgekürzt:

B = BamHI    N = NcoI
Bg = BglII    Ss = SstI
E = EcoRI    X = XbaI
H = HindIII

Bei BglII und NcoI wurden nur die zur Konstruktion des Hybridgens gemäß Figur 2 notwendigen Schnittstellen eingezeichnet.

Die Figur 2 zeigt (nicht maßstabsgerecht) die Konstruktion eines Hybridserpingens mit den Exons 1 bis 4 von hLS2, dargestellt als ausgefüllte Balken und bezeichnet als "Ex 1" bis "Ex 4" wie in Figur 1 und dem 3'-terminalen Exon des humanen $\alpha^1$-Antitrypsingens, dargestellt als schraffierter Balken und bezeichnet als "Ex $\alpha^1$-AT".

Soweit die üblichen Bezeichnungen der Restriktionsenzyme abgekürzt wurden, gelten die Erläuterungen zu Figur 1; zusätzlich wurden verwendet:

P = PstI
S = SalI
Sm = SmaI

Xh = XhoI
K = Auffüllen mit Klenow-Polymerase
S1 = Abbau mit S1-Nuklease
Ph = alkalische Phosphatase
L = Linker
Δ bezeichnet Schnittstellen, die durch Abbau oder Auffüllen der überstehenden Enden stumpfendig gemacht wurden.

Die Tabelle 1 zeigt die DNA-Sequenzen der Exons und der flankierenden Regionen des hLS2-Gens, wobei Intronsequenzen durch Kleinbuchstaben dargestellt sind. Das Signalpeptid und das zur Bildung korrekter 3'-Transkriptenden notwendige Signal AATAAA sind unterstrichen. Die Exon-Intron-Grenzen ergeben sich aus einem Vergleich mit der bekannten hLS2-cDNA. Der Pfeil gibt den 5'-Startpunkt des längsten bisher gefundenen hLS2-cDNA-Klones wieder.

Der Begriff "Hybridserpine" soll im Zusammenhang mit der vorliegenden Erfindung besagen, daß es sich um ein Protein handelt, das aus Aminosäureblöcken besteht, die im wesentlichen Exons von hLS2 und analogen Serpinen mit dem gleichen Genaufbau entsprechen, und eine Proteinase-Inhibitor-Aktivität zeigt. Weiterhin soll der Ausdruck "Hybridserpine" die Naturprodukte ausschließen, die theoretisch durch Kombination von identischen Exons aus unterschiedlichen Quellen erhältlich wären. "Im wesentlichen" soll ausdrücken, daß die gentechnisch erhaltenen Produkte in an sich bekannter Weise modifiziert sein können, indem beispielsweise Aminosäuren zugefügt oder weggelassen oder ausgetauscht werden können. Solche Modifikationen sind beispielsweise durch den Einsatz entsprechender Linker oder Adaptoren möglich.

Bei der erfindungsgemäßen Herstellung des rekombinanten Gens können auch synthetische Genfragmente eingesetzt werden. Dieses Vorgehen hat den Vorteil, daß zusätzliche Schnittstellen für Restriktionsenzyme eingebaut werden können, die zusätzliche Modifikationen der kodierten Aminosäuresequenz erlauben. Auf diesem Wege können beispielsweise auch die aktiven Zentren verändert werden und allgemein Hybridserpine mit geänderter Substrat-Spezifität und/oder Aktivität erzeugt werden.

Bei der Rekombination der Exons werden diese zweckmäßig einschließlich aller notwendigen Sequenzen innerhalb der Introns, die für das Spleißen und andere posttranskriptionale Vorgänge notwendig sind, verwendet. Zum Verknüpfen kann man beispielsweise überhängende DNA-Sequenzen durch Abbau oder Auffüllen stumpfendig machen und so die Exons nach Belieben verbinden, gegebenenfalls unter Einfügung von synthetischen Oligonukleotid-Linkern, die als Substrate für günstige Restriktionsenzyme dienen. Derartige Exon-Module können erfindungsgemäß in einer Ligasereaktion in praktisch beliebiger Kombination, aber in der korrekten relativen Orientierung zueinander und in der richtigen Reihenfolge, zusammengesetzt werden. Die so erhaltenen Hybridgene können, wenn nicht ein Serpin-eigener Promotor verwendet wird, mit einem geeigneten eukaryotischen Promotor und gegebenenfalls mit einem Polyadenylierungssignal verbunden werden und nach Einbringen in die entsprechenden eukaryotischen Zellen dort zur Expression gebracht werden.

Als Wirts-Vektor-Systeme können in an sich bekannter Weise höhere Zellen wie Insekten-oder Säugetier-Zellen Verwendung finden. Solche eukaryotischen Expressions-Systeme sind inzwischen in großer Zahl bekannt. Gegebenenfalls kann auch die gebildete Hybridserpin-mRNA isoliert werden und zur Synthese von cDNA dienen, die dann - nach Anfügen geeigneter Transkriptionssignale - zur Expression in Bakterien oder Hefen verwendet werden kann. Arbeitet man in Hefen, so kann man hefetypisch glykosylierte Proteine erhalten.

Neben der Möglichkeit, Hybridserpine mit veränderter Substrat-Spezifität bzw. Aktivität zu erzeugen, eröffnet die Erfindung einen Zugang zur Herstellung bifunktioneller Proteine, die beispielsweise die Aktivitäten von Angiotensin II und Antitrypsin enthalten und somit neben einer Regulation des Wasserhaushalts einen hemmenden Einfluß auf die Funktion der Elastase ausüben.

Die Erfindung bezieht sich weiterhin auf Diagnostika, die genomische DNA von hLS2 ganz oder teilweise enthalten, zur Identifizierung von Gendefekten im hLS2-Gen bzw. Diagnostizierverfahren, bei denen humane DNA oder RNA enthaltendes Material mit entsprechenden Genproben hybridisiert wird.

Die Erfindung wird durch die folgenden Beispiele näher erläutert. Prozente sind Gewichtsprozente, wenn keine anderen Angaben gemacht sind

Beispiel 1:

Isolierung von hLS2-Cosmiden aus einer humanen Plazenta-Genbank

Nach der Methode von W. Lindenmaier et al. (in: 35. Colloquium Mosbach 1984, "The Impact of Gene Transfer Techniques in Eukaryotic Cell Biology", Springer-Verlag Berlin, Heidelberg 1985) wurde eine Cosmidbank erstellt, wobei die partiell mit MspI gespaltenen genomischen Human-DNA-Fragmente mit dem Vektor pHC79-2cos,TK, der mit ClaI und alkalischer Phosphatase behandelt wurde, verknüpft sind. Die Cosmidbank enthält etwa 300 000 unabhängige Klone. $1,8 \times 10^6$ verpackte Cosmide wurden mit 5 ml TM-Puffer (50 mM Tris-HCl, pH 7,5, 10 mM $MgSO^4$) und 5 ml einer Übernachtkultur von E. coli DH1, die bei 37°C in NZ-Medium (10 g/l NZ-Amin, 5 g/l Kochsalz, 2 g/l $MgCl^2$ 6 $H^2O$, 4 mg/l Thiamin) mit 0,4 % Maltose gewachsen war, gemischt und 20 Minuten bei 37°C ohne Schütteln inkubiert. Nach Zugabe von 40 ml LB-Medium (10 g/1 Bactotrypton (Difco), 5 g/l Hefeextrakt (Difco), 10 g/l Kochsalz) mit 4 mg/l Thiamin wurde die Kultur eine Stunde bei 37°C geschüttelt.

Jeweils 5 ml einer solchen Bakterienkultur wurden auf autoklavierten Nitrocellulosemembranen auf der Oberfläche einer Agarplatte ($23 \times 23$ cm; LB-Medium mit 1,4 % Agar und 50 μg/ml Ampicillin) verteilt. Die Platten wurden bei 37°C inkubiert, bis die Kolonien einen Durchmesser von etwa 0,5 mm aufwiesen. Die Herstellung von Replikafiltern, die Lyse und die Fixierung der Kolonien für die Hybridisierung erfolgte nach bekannten Methoden (D. Hanahan und M. Meselson, Methods in Enzymology 100 (1983) 333-342, T. Maniatis et al., Molecular Cloning, Cold Spring Harbor, 1982). Die Filter wurden eine Stunde bei 42°C mit "prewashing solution" ( Maniatis et al., a.a.O. S. 326) gewaschen. Die Vorhybridisierung (4 bis 6 Stunden) und die Hybridisierung (16 Stunden bei 60°C) wurden in der folgenden Lösung durchgeführt:

0,9 M NaCl
0,18 M Tris-HCl, pH 8,0
$5 \times$ Denhardts Lösung
0,2 % (w/v) SDS (Natriumdodecylsulfat)
200 μg/ml gescherte und erhitzte Kalbsthymus-DNA
200 μg/ml Hefe-RNA
0,5 % (v/v) nichtionisches Detergenz (Nonidet P-40;Sigma)

Als Sonden für die Hybridisierung wurden die folgenden Restriktionsfragmente der hLS2-cDNA (EP-A 0 190 652) eingesetzt:

A) 1,07 kb HindIII-Fragment aus dem Plasmid pH14, das die 5'-Hälfte der hLS2-cDNA umfaßt.

B) 0,5 kb XmrI-Fragment aus dem Plasmid pL10/2. Dieses Fragment ist in der 3'-Hälfte der hLS2-cDNA lokalisiert (Positionen 1121 bis 1619).

Die genannten Fragmente wurden durch Behandlung der jeweiligen Plasmide mit den entsprechenden Restriktions-Enzymen ausgeschnitten, nach Auftrennung auf Agarosegelen elektroeluiert und "nicktranslatiert" ($\geq 10^8$ cpm/μg; Maniatis et al., a.a.O.).

Die Membranen wurden nach der Hybridisierung jeweils 30 Minuten bei Raumtemperatur, bei 45°C und bei 60°C mit $0,1 \times$ SSC ($1 \times$ SSC = 0,15 M NaCl, 15 mM Natriumcitrat, pH 7,0) mit 0,1 % SDS gewaschen.

Nach dem Trocknen wurden mit diesen Membranen Röntgenfilme exponiert. Hybridisierende Kolonien wurden durch Verdünnen vereinzelt und wie beschrieben auf Nitrocellulosemembranen angezüchtet, lysiert und wieder hybridisiert. Nach nochmaliger Vereinzelung verblieben von insgesamt 750 000 analysierten Kolonien 3 unabhängige hybridisierungspositive Klone. Der Klon p4R hybridisierte nur mit der Sonde A, nicht jedoch mit der Sonde B. Umgekehrt hybridisierten die Klone p6R und p9R nur mit der Sonde B, nicht jedoch mit A.

Cosmid-DNA der Klone p4R, p6R und p9R wurde nach der alkalischen Lysemethode (Maniatis et al., a.a.O.) isoliert. Die DNA-Präparationen wurden mit verschiedenen Restriktionsendonukleasen geschnitten, auf 0,7 %igen oder 1 %igen Agarosegelen aufgetrennt und auf Nitrocellulose-Membranen transferiert (E. Southern, J. Molec. Biol. 98 (1975) 503-517). Exon-enthaltende DNA-Fragmente der Cosmide wurden durch Hybridisierung mit verschiedenen radioaktiv markierten Restriktionsfragmenten der hLS2-cDNA identifiziert, die im folgenden aufgeführt sind:

Sonde 1: HindIII-BamHI-Fragment, das den Bereich zwischen den Positionen 1 und 310 der hLS2-cDNA enthält. (Die HindIII-Stelle ist in der Polylinker-Region des Vektors pUC13, in den die hLS2-cDNA kloniert ist, lokalisiert).

Sonde 2: BamHI-Fragment, das die Positionen 311 bis 834 der hLS2-cDNA umfaßt.

Sonde 3: PvuII-Fragment mit dem Bereich zwischen den Positionen 984 und 1399.

Sonde 4: XmnI-Fragment, das den Bereich zwischen den Positionen 1121 und 1619 umfaßt.

Sonde 5: EcoRI-Fragment. das den Bereich zwischen den Positionen 1560 und 2081 umfaßt (eine der EcoRI-Stellen ist in der Polylinker-Region des Vektors pUC13 lokalisiert).

Alle hier angeführten Positionen für Restriktionsenzym-Schnittstellen beziehen sich auf den kodierenden Strang in der cDNA.

Die Hybridisierung erfolgte jeweils bei 42°C über Nacht in dem vorstehend genannten Hybridisierungspuffer. Die Membranen wurden anschließend 2 $\times$ 15 Minuten bei Raumtemperatur in 2 $\times$ SSC mit 0,1 % SDS und dann 2 $\times$ 15 Minuten bei 42°C in 0,1 $\times$ SSC mit 0,1 % SDS gewaschen, getrocknet und auf Röntgenfilme exponiert. Die Membranen wurden zur Entfernung der Proben jeweils 2 Minuten lang in kochendem Wasserbad inkubiert und dann zur Rehybridisierung wieder verwendet.

Zur Identifizierung des ersten Exons (Ex 1) wurde das Oligonukleotid mit der DNA-Sequenz
3′-CGCGGTGAAGAGTCTTTGTGTCTC-5′
(das hier entgegen der üblichen 5′-3′-Richtung wiedergegeben ist) nach dem Phosphoramiditverfahren (M.D. Matteucci et al., J. Am. Chem. Soc. 103 (1981) 3185-3191) synthetisiert und über ein Polyacrylamid-Harnstoff-Gel gereinigt. Die Sequenz des Oligonukleotids wurde aus einer humanen hLS2-cDNA-Sequenz abgeleitet. die aus einer $\lambda$gt10-cDNA-Bank isoliert wurde, die nach der Methode von T. Huynh et al., in: D.M. Glover (Hrsg.), DNA Cloning, a Practical Approach, Vol. I, IRL Press, Oxford UK, Washington, D.C. 1985, Seiten 49-78, gewonnen worden war. Cosmid-DNA des Klons p4R wurde, wie vorstehend beschrieben, mit Hilfe der Southern-Technik analysiert. Das Oligonukleotid mit der vorstehend genannten DNA-Sequenz wurde mit Hilfe von $\lambda$-$^{32}$P-ATP (NEN) und Polynukleotidkinase radioaktiv markiert (Maniatis et al., a.a.O.). Die Hybridisierungstemperatur betrug 42°C.

Das Waschen der Membranen erfolgte mit 6 $\times$ SSC bei Raumtemperatur (2 $\times$ 15 Minuten) bzw. bei 33°C (2 $\times$ 30 Minuten).

Aufgrund der Hybridisierungsexperimente wurden überlappende Restriktionsfragmente der Cosmide nach Standardverfahren in den Vektor pUC13 subkloniert. Die Exons sowie benachbarte Intronbereiche wurden nach der chemischen Abbaumethode von A. Maxam und W. Gilbert, Methods in Enzymology 65 (1980) 499-560, sequenziert. Die Figur 1 zeigt schematisch, den aus Restriktionspaltung und "Southern blotting" sowie Sequenzierung abgeleiteten Aufbau des hLS2-Gens.

Beispiel 2:

Subklonierung von Exons einschließlich flankierender Intronsequenzen des hLS2-Gens und Konstruktion eines hLS2-$\alpha$¹-Antitrypsin-Hybridgens.

Im folgenden wird beispielhaft anhand der Tabelle 2 (und der Figur 2) die Konstruktion eines exprimierbaren Hybridserpingens beschrieben. Selbstverständlich können anstelle der beschriebenen Restriktionsfragmente andere geeignete DNA-Fragmente und andere Kombinationen von DNA-Fragmenten verwendet werden. Insbesondere können anstelle der hLS2-spezifischen Promotorregion andere eukaryotische Promotoren, beispielsweise solche eines anderen Serpingens, verwendet werden.

## Tabelle 2

| Restriktions-fragment | enthält | Modifikation der Enden und Subklonierung |
|---|---|---|
| 1,6 kb NcoI/EcoRI | Exon 1 des hLS2-Gens einschließlich Promotorregion | Auffüllen mit Klenowfragment der DNA-PolymeraseI von E. coli; Subklonierung in den Vektor pUC13, der mit SalI gespalten und mit S1-Nuklease behandelt wurde |
| 1,3 kb XbaI/HindIII | Exon 2 des hLS2-Gens (kodiert u.a. für das Signalpeptid) | Auffüllen mit Klenowfragment, Subklonierung in den Vektor pUC13, der mit BamHI und mit S1-Nuklease behandelt wurde |
| 0,65 kb SstI/SstI | Exon 3 des hLS2 Gens | Spalten mit S1-Nuklease, Subklonierung in den Vektor pUC13, der mit SalI gespalten und mit der S1-Nuklease behandelt wurde |
| 1,0 kb BglII/BamHI | Exon 4 des hLS2-Gens | Auffüllen mit Klenowfragment, Subklonierung in den Vektor pUC13, der mit SmaI und alkalischer Phosphatase behandelt wurde |

Alle Exon-enthaltenden Restriktionsfragmente umfassen gleichzeitig die für ein korrektes Spleißen eventuell notwendigen DNA-Sequenzen an den Exon-Intron-Grenzen (B. Ruskin et al., Cell 38 (1984) 317-331; E. Keller et al., Proc. Natl. Acad. Sci. USA 81 (1984) 7417-7420; B Wieringa et al., Cell 37 (1984) 915-925).

Die Isolation der DNA-Fragmente auf Agarosegelen, die Behandlung der Fragmentenden und die Subklonierung in den Vektor pUC13 erfolgen nach Standardmethoden (Maniatis et al., a.a.O.). Die Orientierung der Inserts wird durch Restriktionsspaltung festgestellt und die Plasmide mit der richtigen Insertionsorientierung werden zur Konstruktion des Hybridserpingens eingesetzt.

Die Verknüpfung der Exons 1 bis 4 des hLS2-Gens in der korrekten Reihenfolge und korrekten Orientierung der Exons zueinander erfolgt ebenfalls nach bekannten Methoden.

Beispiel 3:

Subklonierung des 3'-terminalen Exons des menschlichen α'-Antitrypsin-Gens

Aus einem genomischen Klon mit dem humanen α'-Antitrypsin-Gen wird ein 1.7 kb langes XhoI HindIII-Fragment mit dem 3'-terminalen Exon des Gens (G. Long et al., Biochemistry 23 (1984) 4828-4837; M. Leicht et al., Nature 297 (1982) 655-659), isoliert. Dieses Exon kodiert u.a. für das reaktive Zentrum des Proteins (G. Long et al., a.a.O.; R. Carrell et al., Nature 298 (1982) 329-334). Nach Reparatur der Enden und Anfügen von EcoRI-Linkern

5'CCGAATTCGG 3'

wird das Fragment in den mit EcoRI und alkalischer Phosphatase behandelten Vektor pUC13 einligiert.

Das menschliche α'-Antitrypsin-Gen kodiert für die Aminosäuren Methionin und Serin im reaktiven Zentrum des Proteins. In einer natürlich vorkommenden α'-Antitrypsin-Variante ist der Methionylrest gegen einen Arginylrest ausgetauscht. Dieser Austausch verleiht dem mutierten Protein antithromtotische Eigenschaften (M. Owen et al., New England J. Med. 309 (1983) 694-698). Derartige Mutationen können durch bekannte Verfahren, beispielsweise in vitro-Mutagenese (W. Kramer et al., Nucleic Acids Res. 12 (1984) 9441-9456) eingeführt werden. Solchermaßen modifizierte Exons können natürlich ebenso zur erfindungsgemäßen Konstruktion von Hybridserpin-Genen verwendet werden.

Die Exons 1 bis 4 des hLS2-Gens (Beispiel 2) werden mit dem 3'-terminalen Exon des α'-Antitrypsin-Gens in bekannter Weise verknüpft (Figur 2).

## Ansprüche

1. Hybridserpine, gekennzeichnet durch Aminosäure-Teilsequenzen, die im wesentlichen Exons mit der Genstruktur von humanem Leuserpin-2 (hLS2) entsprechen.

2. Hybridserpine nach Anspruch 1, dadurch gekennzeichnet, daß sie Aminosäure-Teilsequenzen enthalten, die Exons von hLS2, α'-Antitrypsin oder Angiotensinogen entsprechen.

3. Verfahren zur Herstellung von Hybridserpinen, dadurch gekennzeichnet, daß man aus Exons von mindestens zwei Genen, die die Exon-Intron-Struktur von hLS2 aufweisen und für ein Serpin kodieren, ein Gen rekombiniert, das eine hLS2 entsprechende Genstruktur hat, und dieses rekombinierte Gen in einer Wirtszelle exprimiert.

4. Verfahren nach Anspruch 3, dadurch gekennzeichnet, daß die Wirtszelle eine höhere eukaryotische Zelle ist.

5. Rekombinantes Gen mit einer hLS2 entsprechenden Genstruktur, enthaltend Exons von Serpingenen mit einer hLS2 entsprechenden Genstruktur.

6. Gen nach Anspruch 5, bei dem die Exons von Spleißsignalen und Verzweigungsbereichen ("branch points") der zugehörigen Introns flankiert sind.

7. Genomische DNA-Fragmente, enthaltend ein Exon von hLS2.

8. Genomische DNA von hLS2.

9. Wirtszellen, transfiziert mit DNA nach Anspruch 5 bis 7.

10. Arzneimittel, enthaltend ein Hybridserpin nach Anspruch 1 oder 2.

11. Diagnostikum, das genomische DNA nach Anspruch 7 oder 8 ganz oder teilweise enthält.

12. Diagnoseverfahren, dadurch gekennzeichnet, daß humane DNA mit DNA nach Anspruch 7 oder 8 oder entsprechender RNA hybridisiert wird.

Patentansprüche für folgenden Vertragsstaat : GR :

1. Hybridserpine, gekennzeichnet durch Aminosäure-Teilsequenzen, die im wesentlichen Exons mit der Genstruktur von humanem Leuserpin-2 (hLS2) entsprechen.

2. Hybridserpine nach Anspruch 1, dadurch gekennzeichnet, daß sie Aminosäure-Teilsequenzen enthalten, die Exons von hLS2, α'-Antitrypsin oder Angiotensinogen entsprechen.

3. Verfahren zur Herstellung von Hybridserpinen, dadurch gekennzeichnet, daß man aus Exons von mindestens zwei Genen, die die Exon-Intron-Struktur von hLS2 aufweisen und für ein Serpin kodieren, ein Gen rekombiniert, das eine hLS2 entsprechende Genstruktur hat, und dieses rekombinierte Gen in einer Wirtszelle exprimiert.

4. Verfahren nach Anspruch 3, dadurch gekennzeichnet, daß die Wirtszelle eine höhere eukaryotische Zelle ist.

5. Rekombinantes Gen mit einer hLS2 entsprechenden Genstruktur, enthaltend Exons von Serpingenen mit einer hLS2 entsprechenden Genstruktur.

6. Gen nach Anspruch 5, bei dem die Exons von Spleißsignalen und Verzweigungsbereichen ("branch points") der zugehörigen Introns flankiert sind.

7. Genomische DNA-Fragmente, enthaltend ein Exon von hLS2.

8. Genomische DNA von hLS2.

9. Wirtszellen, transfiziert mit DNA nach Anspruch 5 bis 7.

10. Diagnoseverfahren, dadurch gekennzeichnet, daß humane DNA mit DNA nach Anspruch 7 oder 8 oder entsprechender RNA hybridisiert wird.

Patentansprüche für folgeden Vertragsstaat : ES 1. Verfahren zur Herstellung von Hybridserpinen, dadurch gekennzeichnet, daß man aus Exons von mindestens zwei Genen, die die Exon-Intron-Struktur von humanem Leuserpin-2 (hLS2) aufweisen und für ein Serpin kodieren, ein Gen rekombiniert, das eine hLS2 entsprechende Genstruktur hat, und dieses rekombinierte Gen in einer Wirtszelle exprimiert.

2. Verfahren nach Anspruch 1, dadurch gekennzeichnet, daß die Wirtszelle eine eukaryotische Zelle ist.

3. Verfahren nach Anspruch 2, dadurch gekennzeichnet, daß die Wirtszelle eine höhere eukaryotische Zelle ist.

4. Verfahren nach Anspruch 1, 2 oder 3, dadurch gekennzeichnet, daß die Exons für Aminosäure-Teilsequenzen kodieren, die Exons von hLS2, $\alpha^{\cdot}$-Antitrypsin oder Angiotensinogen entsprechen.

5. Verfahren nach einem oder mehreren der vorhergehenden Ansprüche, dadurch gekennzeichnet, daß die Exons von Spleißsignalen und Verzweigungsbereichen ("branch points") der zugehörigen Introns flankiert sind.

FIG. 1

1kb

E B    Ss  H    Bg Ss    H Ss H   Ss  B  H    Ss    Ss B Ss    Bg  B  B    B
X   N        N      E  X    X X   E       X              X X              X        E

Ex1              Ex2         Ex3   Ex4  Ex5

0 283 932

FIG.2

0 283 932

FIG. 2a